# EUROPÄISCHE PATENTANMELDUNG

(11) **EP 4 400 122 A1**
(43) Veröffentlichungstag der Anmeldung: **17.07.2024**
(21) Anmeldenummer: 24150816.7
(22) Anmeldetag: 09.01.2024
(51) Int. Cl.: A61L 2/18

(54) **SYSTEM ZUR FÖRDERUNG EINES STERILISATIONSMEDIUMS UND VERFAHREN ZUR BEREITSTELLUNG EINES STERILISATIONSMEDIUMS**

(30) Priorität: 16.01.2023 DE 102023100887
(71) Anmelder: Bürkert Werke GmbH & Co. KG, 74653 Ingelfingen (DE)
(72) Erfinder: GUNNESCH, Johann, 74653 Ingelfingen (DE); IWASCHKIN, Alexej, 74653 Ingelfingen (DE); SCHÄFER, Harald, 74653 Ingelfingen (DE)
(74) Vertreter: Prinz & Partner mbB

(57) **Zusammenfassung**

Ein System (10) zur Förderung eines Sterilisationsmediums (12) umfasst zumindest zwei schaltungstechnisch parallel angeordnete Pufferbehälter (14) für Sterilisationsmedium (12), zumindest einen Flüssigkeitsdurchflussregler (32), mit dem die Pufferbehälter (14) jeweils fluidisch gekoppelt sind, und eine Druckmedienversorgungsvorrichtung (56), die eine Druckmediumquelle (40) mit den Pufferbehältern (14) koppelt und eingerichtet ist, die Pufferbehälter (14) mit einem Druckmedium (41) zu beaufschlagen. Das System (10) ist derart ausgebildet, dass für jeden Pufferbehälter (14) ein Füllmodus und ein Sprühmodus einstellbar sind. Im Füllmodus ist der entsprechend geschaltete Pufferbehälter (14) mit dem Sterilisationsmedium (12) aus einer Versorgungsvorrichtung (22) befüllbar und im Sprühmodus ist der entsprechend geschaltete Pufferbehälter (14) fluidisch mit dem Flüssigkeitsdurchflussregler (32) gekoppelt und ist das Sterilisationsmedium (12) aus diesem Pufferbehälter (14) in Richtung des Flüssigkeitsdurchflussreglers (32) basierend auf einem von dem Druckmedium (41) ausgeübten Druck förderbar. Die Pufferbehälter (14) sind wechselweise schaltbar.

## Beschreibung

Die vorliegende Erfindung betrifft ein System zur Förderung eines Sterilisationsmediums und ein Verfahren zur Bereitstellung eines Sterilisationsmediums.

Lebensmittelverpackungen und andere Objekte werden heutzutage vielfach mittels Sterilisationsmedien (beispielsweise Wasserstoffperoxid) beaufschlagt, um diese von Keimen und Bakterien zu befreien. Da die Sterilisationsmedien oftmals aggressiv sind (beispielsweise ätzend), werden dabei spezialisierte Peristaltikpumpen zur Förderung eingesetzt. Aufgrund des Kontakts mit dem Sterilisationsmedium sind diese Peristaltikpumpen aber besonders wartungsintensiv, wodurch der Betriebsaufwand erhöht ist.

Zusätzlich verursachen Peristaltikpumpen eine Pulsation des geförderten Sterilisationsmediums, wodurch eine erhöhte Ausgasung des Sterilisationsmediums bewirkt wird. Ferner wird aufgrund der Pulsation ein gleichmäßiger, kontinuierlicher Durchlauf des Sterilisationsmediums verhindert. Insbesondere wird die Durchflussmessung und Durchflussregelung nachteilig beeinflusst, wodurch auch die Präzision des Sterilisationsprozesses reduziert ist.

Zudem setzen bekannte Systeme oftmals Puffertanks ein, die nach dem Verbrauch der jeweiligen gepufferten Menge des Sterilisationsmediums wieder erneut befüllt werden müssen. Dadurch werden zusätzliche Unterbrechungen des Sterilisationsprozesses verursacht.

Als Alternative sind auch Ringkreissysteme bekannt, bei denen ein zu sterilisierendes Objekt mit dem Sterilisationsmedium beaufschlagt wird. Anschließend wird das Sterilisationsmedium durch eine Ringkreistopologie des Versorgungskreises zurückgewonnen. Allerdings verursachen solche Ringkreissysteme ebenfalls erhöhte Ausgasungen des Sterilisationsmediums aus dem System und erfordern komplexe Filtervorrichtungen, um eine vorbestimmte Konzentration des Sterilisationsmediums zu gewährleisten.

Der Erfindung liegt daher die Aufgabe zugrunde, die Nachteile des Stands der Technik auszuräumen oder zumindest zu verringern. Insbesondere besteht ein Bedürfnis dafür, ein System und ein Verfahren bereitzustellen, mittels denen eine kontinuierliche, unterbrechungsfreie Dosierung eines Sterilisationsmediums ermöglicht wird.

Die Aufgabe wird durch die Gegenstände der unabhängigen Patentansprüche gelöst. Vorteilhafte Ausgestaltungen sind in den abhängigen Patentansprüchen und der nachfolgenden Beschreibung angegeben, von denen jeder für sich oder in (Sub-)Kombination Aspekte der Erfindung darstellen kann. Einzelne Aspekte der Erfindung werden in Bezug auf Vorrichtungen, andere in Bezug auf Verfahren erläutert. Die Aspekte sind aber jeweils wechselseitig entsprechend zu übertragen.

Gemäß einem Aspekt wird ein System zur Förderung eines Sterilisationsmediums bereitgestellt. Das System umfasst zumindest zwei schaltungstechnisch parallel angeordnete Pufferbehälter zur Speicherung des Sterilisationsmediums, zumindest einen Flüssigkeitsdurchflussregler, mit dem die Pufferbehälter jeweils fluidisch gekoppelt sind, und eine Druckmedienversorgungsvorrichtung. Die Druckmedienversorgungsvorrichtung koppelt eine Druckmediumquelle mit den Pufferbehältern und ist eingerichtet, die Pufferbehälter mit einem Druckmedium zu beaufschlagen. Das System ist derart ausgebildet, dass für jeden Pufferbehälter ein Füllmodus und ein Sprühmodus einstellbar sind. Im Füllmodus ist der entsprechend geschaltete Pufferbehälter mit dem Sterilisationsmedium aus einer Versorgungsvorrichtung befüllbar. Im Sprühmodus ist der entsprechend geschaltete Pufferbehälter fluidisch mit dem Flüssigkeitsdurchflussregler gekoppelt und das Sterilisationsmedium aus diesem Pufferbehälter in Richtung des Flüssigkeitsdurchflussreglers basierend auf einem von dem Druckmedium ausgeübten Druck förderbar. Die Pufferbehälter sind wechselweise schaltbar, so dass während des Sprühmodus des einen Behälters der andere oder ein anderer Pufferbehälter im Füllmodus ist.

Das vorliegende System stellt eine Vielzahl an Vorteilen bereit. Das System erfordert vorteilhafterweise keine Pumpen zur Förderung des Sterilisationsmediums, es ist bezüglich des Sterilisationsmediums pumpenlos. Die Förderung des Sterilisationsmediums wird anhand eines Druckpolsters ermöglicht, das durch das Druckmedium bereitgestellt wird. Dadurch kann die Betriebseffizienz gesteigert werden, denn die wartungsintensiven Spezialpumpen für derartige Sterilisationsmedien entfallen.

Durch den Verzicht auf Peristaltikpumpen wird die Förderung des Sterilisationsmediums zudem pulsationsfrei. Somit wird ein kontinuierlicher, gleichmäßiger Durchlauf des Sterilisationsmediums ermöglicht. Insbesondere ist dadurch auch die Präzision der Durchflussmengenbestimmung und Durchflussregelung verbessert, da das Sterilisationsmedium mit einem gleichbleibenden Volumenstrom bereitstellbar ist.

Durch die Vermeidung einer Pulsation (keine Druckstöße) geht ferner eine geringere Ausgasung des Sterilisationsmediums aus dem System einher, so dass die Verluste des Sterilisationsmediums reduziert sind.

Außerdem ist die Flexibilität der Bereitstellung des Sterilisationsmediums verbessert. Peristaltikpumpen für derartige Sterilisationsmedien weisen in der Regel feststehende Förderparameter auf. Das bedeutet, dass das Sterilisationsmedium nur mit feststehenden Durchflussmengen bereitgestellt werden kann. Im Gegensatz dazu kann vorliegend durch die Nutzung eines Druckpolsters die Durchflussmenge des Sterilisationsmediums bedarfsgerecht angepasst werden. Dadurch wird eine Dosierung des Sterilisationsmediums über einen weiten Parameterbereich ermöglicht, je nach den Anforderungen der nachgelagerten Durchflussregelung.

Dadurch, dass das System zwei Pufferbehälter umfasst, die wahlweise zwischen dem Füllmodus und dem Sprühmodus umgeschaltet werden können, kann insbesondere von einem Pufferbehälter, für den der Sprühmodus eingestellt ist, unterbrechungsfrei auf den zweiten Pufferbehälter im Sprühmodus umgeschaltet werden. Dadurch wird eine kontinuierliche Dosierung des Sterilisationsmediums ermöglicht. In anderen Worten kann während des Betriebs des Systems optional immer für zumindest einen Pufferbehälter der Sprühmodus eingestellt sein.

Einzig während der Umschaltung von einem Pufferbehälter im Sprühmodus zu einem anderen Pufferbehälter im Sprühmodus kann das System einen einmaligen vernachlässigbaren kurzen Störimpuls zeigen. Während der Bereitstellung des Sterilisationsmediums aus einem jeweiligen Pufferbehälter zeigt das System im Betrieb allerdings keine Pulsation. In diesem Sinne, kann das System auch als quasi-pulsationsfrei bezeichnet werden.

Das System ist vorliegend auch nicht als Ringkreislauf ausgelegt, bei dem das Sterilisationsmedium zurückgewonnen wird. Auch diesbezüglich ist die Ausgasung des Sterilisationsmediums aus dem System gegenüber bekannten Ringkreissystemen deshalb reduziert. Ferner können komplexe Filtervorrichtungen vermieden werden, die gemäß bekannter Ringkreissysteme benötigt werden, um die Reinheit und Konzentration des Sterilisationsmediums zu gewährleisten.

Zusätzlich ist das System modular je nach den Bedürfnissen erweiterbar. Beispielsweise können zusätzliche Pufferbehälter oder Aerosolausgänge vorgesehen sein. Ferner kann das Tankvolumen eines oder mehrerer Pufferbehälter bedarfsgerecht angepasst werden.

Optional können auch mehrere Flüssigkeitsdurchflussregler vorgesehen sein, mit denen jeweils die parallel angeordneten Pufferbehälter fluidisch gekoppelt sind. So können definierte Fluidströme bedarfsgerecht für unterschiedliche Aerosolausgänge bereitgestellt werden.

Das System ermöglicht daher ein hohes Maß an Flexibilität, eine Reduzierung der Ausgasung des Sterilisationsmediums und eine vorteilhaft verbesserte gleichmäßigere Bereitstellung des Sterilisationsmediums anhand eines Druckpolsters und eine Vermeidung einer Pulsation. Somit ist sowohl die Durchflussmengenbestimmung als auch die Durchflussregelung mit erhöhter Präzision möglich.

Gemäß einem weiteren Aspekt wird auch ein Verfahren zur Bereitstellung eines Sterilisationsmediums anhand eines Systems wie zuvor beschrieben bereitgestellt. Sofern für einen Pufferbehälter der Füllmodus eingestellt ist, wird dieser Pufferbehälter mit dem Sterilisationsmedium aus einer Versorgungsvorrichtung befüllt. Sofern für einen Pufferbehälter der Sprühmodus eingestellt ist, wird dieser Pufferbehälter mit dem Druckmedium ausgehend von der Druckmedienversorgungsvorrichtung derart beaufschlagt, dass ein Druck im Pufferbehälter erzeugt wird. Dann ist dieser Pufferbehälter zudem mit dem Flüssigkeitsdurchflussregler fluidisch gekoppelt. Ferner wird basierend auf dem durch das Druckmedium im Pufferbehälter erzeugten Druck Sterilisationsmedium aus diesem Pufferbehälter in Richtung des Flüssigkeitsdurchflussreglers gefördert. Das System ist derart schaltbar, dass während des Betriebs des Systems immer für zumindest einen Pufferbehälter der Sprühmodus einstellbar ist.

Die Vorteile, die durch das erfindungsgemäße System erzielt werden, werden gleichfalls in entsprechender Weise durch das Verfahren erzielt.

Unter einem Sterilisationsmedium ist vorliegend ein Medium zu verstehen, dass nutzbar ist, um ein Objekt von Keimen und Bakterien bzw. lebenden Organismen zu befreien. Optional ist das Sterilisationsmedium flüssig.

Unter der Druckmedienversorgungsvorrichtung kann vorliegend ein Teil des Systems, eine komplexe Vorrichtung oder ein Subsystem verstanden werden, dass eingerichtet ist, um ein Druckmedium bereitzustellen. Die Druckmedienversorgungsvorrichtung kann dazu eigene Ventile, Speicherreservoirs, Förderleitungen und andere drucktechnisch relevante Komponenten aufweisen.

Unter dem Druckmedium kann vorliegend ein Medium verstanden werden, das geeignet ist, um bezüglich des in den Pufferbehältern bevorrateten Sterilisationsmediums ein Druckpolster zu erzeugen. Das Druckmedium kann in den Pufferbehältern flüssig oder gasförmig sein, bevorzugt gasförmig.

Optional kann das Druckmedium derart sein, dass keine Durchmischung mit dem Sterilisationsmedium erfolgt, oder nur eine vernachlässigbare Durchmischung.

In einigen Ausführungsformen kann das Druckmedium insbesondere Druckluft umfassen.

Der Flüssigkeitsdurchflussregler kann eingerichtet sein, um einen Volumenstrom des Sterilisationsmediums entsprechend einem vorgegebenen Sollwert einzuregeln.

Die Versorgungsvorrichtung kann vorliegend als eine übergeordnete Vorrichtung oder ein übergeordnetes System verstanden werden, aus dem die einzelnen Pufferbehälter mit dem Sterilisationsmediums befüllbar sind. Zum Beispiel kann die Versorgungsvorrichtung einen Haupttank für das Sterilisationsmedium aufweisen, an den das System bezüglich des Sterilisationsmediums gekoppelt ist. Zudem kann die Versorgungsvorrichtung auch ein Versorgungsnetz aufweisen, über das das Sterilisationsmedium bereitstellbar ist.

Bevorzugt sind die Pufferbehälter auch derart schaltbar, dass sie gleichzeitig befüllbar sind, sich also gleichzeitig im Füllmodus befinden. Dies ist beispielsweise bei einer Initialfüllung des Systems vorteilhaft, da dann gleichzeitig für alle Pufferbehälter nominale Sollfüllstände des Sterilisationsmediums erreicht werden können.

Optional herrscht im Pufferbehälter, für den der Füllmodus eingestellt ist, Atmosphärendruck. Dadurch wird die Flexibilität des Systems weiter verbessert. Da die Pufferbehälter gegen Atmosphärendruck gefüllt werden, kann jeder Pufferbehälter je nach den Bedürfnissen entsprechend einem vorgewählten Füllstand befüllt werden. Beispielsweise kann der Füllvorgang dadurch auch basierend auf Ausnutzung der geodätischen Höhe erfolgen. Zudem muss das Sterilisationsmedium nicht unter Druck in den jeweiligen Pufferbehälter befüllt werden.

In einigen Ausführungsformen ist jeder Pufferbehälter jeweils mit einer mechanischen oder elektronischen Druckmessvorrichtung gekoppelt. Insbesondere kann die Druckmessvorrichtung eingerichtet sein, um den Fluiddruck im jeweiligen Pufferbehälter zu bestimmen. Die Information kann genutzt werden, um eine Automatisierung der Förderung des Sterilisationsmediums bereitzustellen.

Bevorzugt kann die Druckmessvorrichtung alternativ oder kumulativ ebenfalls eingerichtet sein, um den Gasdruck über einer Fluidmenge im jeweiligen Pufferbehälter zu bestimmen. Der Gasdruck kann dabei beispielsweise von dem Druckmedium ausgeübt werden.

Optional kann die Druckmessvorrichtung auch einen Drucktransmitter umfassen oder damit gekoppelt sein. Dann kann der jeweils bestimmte Druck anhand des Drucktransmitters zu einer übergeordneten Steuervorrichtung des Systems übermittelt werden. Dadurch wird die Regelung bezüglich der Förderung des Sterilisationsmediums und des Druckmediums vereinfacht.

Bevorzugt weist jeder Pufferbehälter zumindest einen Füllstandsmesser auf. Der Füllstandsmesser kann ferner eingerichtet sein, um den jeweils bestimmten Füllstand an eine Steuervorrichtung des Systems zu übermitteln. Basierend auf dem Füllstand des jeweiligen Pufferbehälters wird die Regelung der Förderung des Sterilisationsmediums vereinfacht.

In einigen Ausführungsformen weist das System ferner eine mechanische oder elektronische Druckregelungsvorrichtung auf. Die Druckregelungsvorrichtung ist zumindest mit den Pufferbehältern und der Druckmedienversorgungsvorrichtung gekoppelt und eingerichtet, jeden Pufferbehälter mit einem vordefinierten Druck basierend auf dem Druckmedium zu beaufschlagen. Insbesondere kann der vordefinierte Druck, der durch die Druckregelungsvorrichtung bezüglich des Druckmediums in dem jeweiligen Pufferbehälter gewährleistet wird, variabel sein. Da das durch das Druckmedium bereitgestellte Druckpolster die Förderung des Sterilisationsmediums gewährleistet, kann so auf vorteilhaft einfache Weise eine variable Fördermenge des Sterilisationsmediums bereitgestellt werden.

Optional ist die Druckregelungsvorrichtung zumindest mittelbar mit einer Druckmessvorrichtung des jeweiligen Pufferbehälters gekoppelt. Beispielsweise können sowohl die Druckmessvorrichtung als auch die Druckregelungsvorrichtung mit einer übergeordneten Steuervorrichtung des Systems gekoppelt sein. Die Steuervorrichtung steuert die Druckregelungsvorrichtung und damit die Förderung des Druckmediums derart, dass ein gewünschter Druck des Druckmediums in dem jeweiligen Pufferbehälter als Druckpolster oberhalb des Sterilisationsmediums gewährleistet wird.

Die Druckregelung kann auch als Computerprogramm anhand der Steuervorrichtung ausgeführt werden, die dazu eine entsprechende Datenverarbeitungsvorrichtung aufweisen kann und einen Druckregler entsprechend steuert.

Bevorzugt weist das System zwischen der Druckregelungsvorrichtung und den Pufferbehältern für jeden Pufferbehälter ein Proportionalventil auf. Dadurch kann ein Regelkreis, mittels dem das Druckpolster anhand des Druckmediums oberhalb des Sterilisationsmediums innerhalb eines Pufferbehälter gewährleistet wird, komplexere Regelmechanismen berücksichtigen. Durch ein Proportionalventil wird beispielsweise eine PID-Regelung (Proportional-Integral-Differential)-Regelung ermöglicht. Das Regelverhalten wird dadurch verbessert. Beispielsweise kann der Regelmechanismus durch eine übergeordnete Steuervorrichtung des Systems ausgeführt werden, die das Proportionalventil und andere Ventile bzw. Komponenten des Systems entsprechend steuert.

Optional kann zwischen der Druckregelungsvorrichtung und den Pufferbehältern für jeden Pufferbehälter auch statt eines Proportionalventils ein Absperrventil (Schaltventil; binär wirkendes Ventil) angeordnet sein. Auch ein Absperrventil kann ausreichend sein, um das Druckpolster anhand des Druckmediums oberhalb des Sterilisationsmediums innerhalb eines jeweiligen Pufferbehälter zu gewährleisten.

In einigen Ausführungsformen kann zwischen der Druckregelungsvorrichtung und den Pufferbehälter jeweils lediglich ein Proportionalventil angeordnet sein. Optional, beispielsweise um zusätzliche Sicherheitsmechanismen zu berücksichtigen, kann neben dem Proportionalventil auch ein zusätzliches Ventil, beispielsweise eine 3/2-Wegeventil oder ein Absperrventil, zwischen der Druckregelungsvorrichtung und den jeweiligen Pufferbehältern angeordnet sein. Dadurch kann das Regelungskonzept bezüglich der Förderung des Druckmediums und damit auch des Sterilisationsmediums bedarfsgerecht angepasst werden.

Optional ist jeder Pufferbehälter jeweils mit zumindest einem Druckentlastungsventil gekoppelt. Da sich das Sterilisationsmedium beim Übergang in den gasförmigen Aggregatzustand ausdehnt, wird so ein zusätzlicher Sicherheitsmechanismus bereitgestellt.

Bevorzugt ist das Druckentlastungsventil derart eingerichtet, dass in dem jeweiligen Pufferbehälter bei geöffnetem Entlüftungsventil Atmosphärendruck herrscht. Dadurch wird der Füllvorgang anhand des Druckentlastungsventils vereinfacht, da dann gegen Atmosphärendruck gefüllt werden kann.

In einigen Ausführungsformen ist zwischen jedem Pufferbehälter und der Druckmedienversorgungsvorrichtung ein Rückschlagventil derart angeordnet, dass ein Rückfluss vom Pufferbehälter in die Druckmedienversorgungsvorrichtung unterbunden wird. Dadurch wird eine Verunreinigung der Druckmedienversorgungsvorrichtung mit dem Sterilisationsmedium verhindert. Ferner kann so ein Solldruck des Druckpolsters, das anhand des Druckmediums erzeugt wird, welches von der Druckmedienversorgungsvorrichtung bereitgestellt wird, in einfacher Weise gewährleistet werden.

Optional weist das System ferner zumindest eine Zweistoffdüse auf, die ausgehend von dem Flüssigkeitsdurchflussregler mit dem Sterilisationsmedium und zusätzlich mit einem Fördermedium beaufschlagbar ist. Die Zweistoffdüse ist eingerichtet, das Sterilisationsmedium durch das Fördermedium zu zerstäuben. Dadurch kann das Sterilisationsmedium für eine Endanwendung bereitgestellt werden. Vorteilhaft kann der durch die Zweistoffdüse bereitgestellte zerstäubte Volumenstrom kontinuierlich und gleichmäßig gewährleistet werden.

Alternativ kann die Zweistoffdüse auch extern zum System sein. In diesem Fall wird stromabwärts bezüglich des Flüssigkeitsdurchflussreglers lediglich der voreingestellte Volumenstrom des Sterilisationsmediums bereitgestellt.

In einigen Ausführungsformen können auch mehrere Zweistoffdüsen vorgesehen sein, die ausgehend von dem oder mehreren Flüssigkeitsdurchflussreglern mit dem Sterilisationsmedium und zusätzlich mit einem Fördermedium beaufschlagbar sind.

Bevorzugt kann als Fördermedium, welches von der Zweistoffdüse genutzt wird, um das Sterilisationsmedium zu zerstäuben, ein Medium genutzt werden, das ebenfalls als Druckmedium des Systems nutzbar ist. Beispielsweise kann Druckluft sowohl als Druckmedium als auch als Fördermedium verwendet werden. In diesem Fall kann die Druckmedienversorgungsvorrichtung derart sein, dass das Druckmedium als Fördermedium zusätzlich für die Zweistoffdüse bereitgestellt wird.

Optional weist das System für jeden Pufferbehälter ferner eine Spülvorrichtung auf, die eingerichtet ist, um den jeweiligen Pufferbehälter mit einem Reinigungsmittel zu spülen.

Bevorzugt kann das Reinigungsmittel beispielsweise Druckluft oder Wasser umfassen, beispielsweise destilliertes Wasser. Optional kann das Reinigungsmittel auch Wasser mit Reinigungszusätzen umfassen.

In einigen Ausführungsformen hat jeder Pufferbehälter eine Auslassleitung, die zu einer gemeinsamen Leitung zum Flüssigkeitsdurchflussregler führt. In den jeweiligen Auslassleitungen sind Absperrventile vorgesehen, die im Füllmodus geschlossen sind. Dadurch kann jeder Pufferbehälter bedarfsgerecht für den Füllmodus vom Flüssigkeitsdurchflussregler abgekoppelt werden.

Optional ist zumindest eines der Absperrventile, die in den jeweiligen Auslassleitungen angeordnet sind, als "normal offen" ausgebildet. Das bedeutet, dass ohne Bereitstellung eines Regelungssignals, beispielsweise einer Steuerspannung oder der Beaufschlagung mit einem Steuerfluid oberhalb eines vorbestimmten Grenzwerts, das Ventil offen ist. Wird der jeweilige Grenzwert überschritten, so sperrt das Ventil. Dadurch wird ein zusätzlicher Sicherheitsmechanismus bereitgestellt.

Bevorzugt sind zumindest einige Ventile als pneumatisch betätigbare Ventile ausgebildet. Insbesondere können die pneumatisch betätigbaren Ventilen auch mit dem Druckmedium beaufschlagt werden, um deren Stellung einzustellen. Dann ist vorteilhafter Weise lediglich eine einzelne zusätzliche Druckmedienversorgungsvorrichtung notwendig, um sowohl das Druckmedium zur Bereitstellung des Druckpolsters, um das Fördermedium für die Zweistoffdüse und auch um ein Medium bereitzustellen, mittels dem die Ventile gesteuert werden können.

Optional weist das System eine Steuervorrichtung auf. Die Steuervorrichtung ist zumindest eingerichtet, um die Pufferbehälter entsprechend des Füllmodus oder des Sprühmodus einzustellen. Ferner ist die Steuervorrichtung eingerichtet, Komponenten des Systems entsprechend zu steuern, damit der Füllmodus oder der Sprühmodus eines Pufferbehälters gewährleistet wird. Beispielsweise kann die Steuervorrichtung entsprechende Ventile ansteuern. Ferner kann die Steuervorrichtung auch mit anderen Komponenten des Systems zu Regelungszwecken gekoppelt sein, beispielsweise Druckmessvorrichtungen, Drucktransmittern oder Druckreglern. Ferner kann die Steuervorrichtung auch eingerichtet sein, um die Förderung des Druckmediums bedarfsgerecht zu gewährleisten. Beispielsweise kann die Steuervorrichtung auch die Steuerung der Druckmedienversorgungsvorrichtung gewährleisten.

In einigen Ausführungsformen ist mit jedem Pufferbehälter zumindest ein Überdruckventil gekoppelt. Dadurch wird ein zusätzlicher Sicherheitsmechanismus geschaffen. Das jeweilige Überdruckventil kann als mechanisches Überdruckventil ausgebildet sein. Dadurch wird der Sicherheitsmechanismus auch bei Ausfall des zugrundeliegenden elektronischen Regelungssystems gewährleistet. Insbesondere ist so die Gefahr eines sich expandierenden Sterilisationsmediums gebannt.

Bevorzugt sind die Auslassleitungen der jeweiligen Pufferbehälter mit einer Drainage zur Entsorgung des Sterilisationsmediums gekoppelt. Das bedeutet, dass das System nicht als Ringkreislauf ausgebildet ist. Dadurch wird die Ausgasung des Sterilisationsmediums aus dem System reduziert. Zusätzlich kann ein jeweiliger Pufferbehälter so, zum Beispiel zu Wartungszwecken, entleert werden.

Optional ist der Flüssigkeitsdurchflussregler unmittelbar mit der Zweistoffdüse gekoppelt. Das bedeutet, dass der Flüssigkeitsdurchflussregler und die Zweistoffdüse auch in einer Vorrichtung kombiniert sein können.

In einigen Ausführungsformen weist das System zusätzliche Pufferbehälter auf, die mit einem der zumindest zwei Pufferbehältern fluidisch parallel geschaltet sind. Eine Gruppe von Pufferbehältern kann dann ein einzelnes zusammenhängendes Volumen (Speichereinheit) für das Sterilisationsmedium bereitstellen. Einige Pufferbehälter sind also zu einem einzelnen zusammenhängenden Volumen zusammengeschaltet. Es gibt aber zumindest zwei Gruppen von Pufferbehältern bzw. eine Gruppe von Pufferbehältern und einen einzelnen Pufferbehälter, für die getrennt der Füllmodus oder der Sprühmodus einstellbar ist.

Alternativ oder kumulativ können auch zusätzliche Pufferbehälter vorgesehen sein, für die zusätzlich, parallel zu den bereits vorhandenen Pufferbehältern, der Füllmodus oder der Sprühmodus einstellbar ist.

Optional können einzelne Pufferbehälter auch separat abgekoppelt werden, beispielsweise zu Wartungszwecken.

Dadurch kann das Gesamtvolumen für das Sterilisationsmedium bedarfsgerecht angepasst werden, beispielsweise in Abhängigkeit der Anlagengröße oder des Anwendungsfalls.

Optional weist das System auch Drucksensoren auf. Beispielsweise kann mit jedem Pufferbehälter ein Drucksensor gekoppelt sein. Ferner kann ein Drucksensor mit einer Zuleitung gekoppelt sein, die zwischen der Versorgungsvorrichtung und den jeweiligen Pufferbehälter angeordnet ist. Der Drucksensor kann auch ausgebildet sein, um eine Druckdifferenz zu bestimmen, beispielsweise in Bezug auf die Zuleitungen, mit denen unterschiedliche Pufferbehälter gekoppelt sind. Dadurch kann die Regelung der Förderung des Sterilisationsmediums verbessert werden.

Insbesondere kann eine Druckmessung für den Eingang des Systems bezüglich der Versorgungsvorrichtung des Sterilisationsmediums vorgesehen sein und/oder für den Eingang der Druckmedienversorgungsvorrichtung bezüglich des Druckmediums. Also können sowohl für den Sterilisationsstrang als auch für den Druckmedienstrang Druckmessungen vorgesehen sein. Die Regelung für die Förderung des Druckmediums und des Sterilisationsmediums wird dadurch vereinfacht.

Optional ist mit jedem Pufferbehälter auch ein Sensor gekoppelt, der eingerichtet ist, um eine Konzentration des Sterilisationsmediums in dem jeweiligen Pufferbehälter zu bestimmen. Dadurch kann gewährleistet werden, dass das Sterilisationsmedium mit der erforderlichen Konzentration vorliegt, bevor es anschließend für eine Endanwendung bereitgestellt wird.

Bevorzugt umfasst das Sterilisationsmedium Wasserstoffperoxid (H2O2). Wasserstoffperoxid ist gut geeignet, um Objekte von Keimen oder Bakterien zu befreien.

Die Erfindung sowie weitere vorteilhafte Ausführungsformen und Weiterbildungen derselben werden im Folgenden anhand des in der Zeichnung dargestellten Beispiels näher beschrieben und erläutert. Die der Beschreibung und der Zeichnung zu entnehmenden Merkmale können einzeln für sich oder zu mehreren in beliebiger Kombination erfindungsgemäß angewandt werden. Es zeigt:
- Fig. 1 eine schematische Darstellung eines erfindungsgemäßen Systems zur Förderung eines Sterilisationsmediums, und
- Fig. 2 eine schematische Darstellung eines erfindungsgemäßen Verfahrens zur Bereitstellung eines Sterilisationsmediums.

Alle nachstehend in Bezug auf die Ausführungsbeispiele und/oder die begleitenden Figuren offengelegten Merkmale können allein oder in einer beliebigen Unterkombination mit Merkmalen der Aspekte der vorliegenden Offenbarung, einschließlich Merkmalen bevorzugter Ausführungsformen, kombiniert werden, vorausgesetzt, die sich ergebende Merkmalskombination ist für einen Fachmann auf dem Gebiet der Technik sinnvoll.

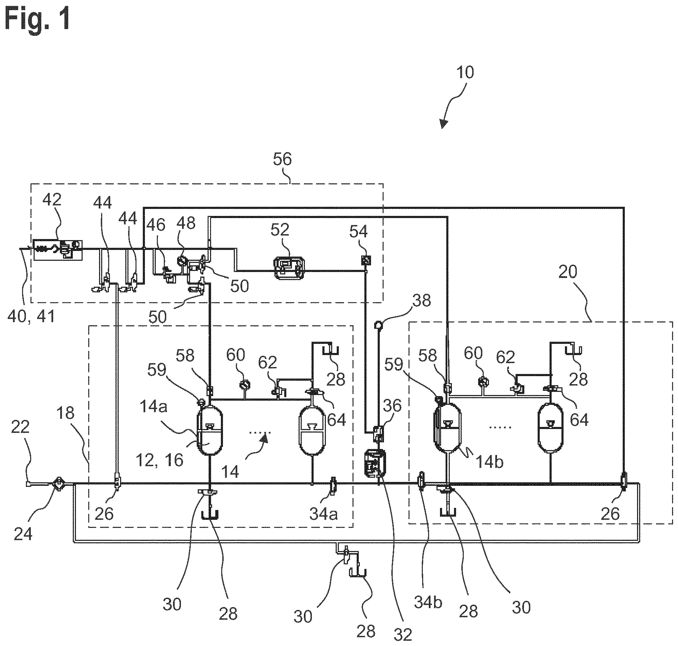

Fig. 1 zeigt eine schematische Darstellung eines erfindungsgemäßen Systems 10 zur Förderung eines Sterilisationsmediums 12, hier von Wasserstoffperoxid.

Das System 10 umfasst mehrere Pufferbehälter 14, jedoch zumindest einen ersten Pufferbehälter 14a und einen zweiten Pufferbehälter 14b.

Jeder Pufferbehälter 14a, 14b weist ein Behältervolumen 16 auf, das zur Speicherung des Sterilisationsmediums 12 eingerichtet ist.

Der erste Pufferbehälter 14a ist einer ersten Speichereinheit 18 und der zweite Pufferbehälter 14b ist einer zweiten Speichereinheit 20 des Systems 10 zugeordnet. Vorliegend umfasst jede Speichereinheit 18, 20 mehrere Pufferbehälter 14, was durch die Punkte angedeutet ist. Gemäß dieser Ausführungsform sind die Pufferbehälter 14 einer Speichereinheiten 18, 20 fluidisch zusammengeschaltet, sodass sie ein entsprechendes einziges Gesamtvolumen für das Sterilisationsmedium 12 der jeweiligen Speichereinheit 18, 20 ausbilden. Die Speichereinheiten 18, 20 müssen jedoch generell lediglich jeweils einen einzelnen Pufferbehälter 14 (vorliegend 14a, 14b) aufweisen.

Innerhalb des Systems 10 sind die Speichereinheiten 18, 20 und deren Pufferbehälter 14a, 14b zueinander schaltungstechnisch parallel geschaltet.

Das System 10 ist mit einer Sterilisationsmedienquelle 22 gekoppelt. Ausgehend von der Sterilisationsmedienquelle 22 kann das System mit dem Sterilisationsmedium 12 befüllt werden. Eingangsseitig weist das System 10 daher eine Filtervorrichtung 24 auf, die zwischen der Sterilisationsmedienquelle 22 und den restlichen Komponenten des Systems 10 angeordnet ist. Die Filtervorrichtung 24 ist eingerichtet, um Fremdstoffe aus dem Volumenstrom des Sterilisationsmediums 12 ausgehend von der Sterilisationsmedienquelle 22 zu entfernen.

Die nachfolgenden technischen Eigenschaften des Systems 10 werden anhand einer einzelnen Speichervorrichtung 18 und eines einzelnen Pufferbehälters 14 davon erläutert, sind aber entsprechend auf die weitere Speichervorrichtung 20 bzw. weitere Pufferbehälter 14 zu übertragen.

Zwischen der Filtervorrichtung 24 und dem Pufferbehälter 14 der Speichervorrichtung 18 ist ein Absperrventil 26 angeordnet. Ist das Absperrventil 26 geschlossen, kann der Pufferbehälter 14 der Speichervorrichtung 18 ausgehend von der Sterilisationsmedienquelle 22 nicht mit dem Sterilisationsmedium 12 befüllt werden. In offener Stellung des Absperrventils 26 ist der Füllvorgang des Pufferbehälter 14 hingegen möglich.

Der Pufferbehälter 14 ist ferner mit einer Drainage 28 gekoppelt, wobei zwischen dem jeweiligen Pufferbehälter 14 und der Drainage 28 ein zusätzliches Absperrventil 30 angeordnet ist. Anhand der Drainage 28 kann ein Pufferbehälter 14 entleert werden, beispielsweise zu Wartungszwecken.

Das System 10 umfasst ferner einen Flüssigkeitsdurchflussregler 32. Der Flüssigkeitsdurchflussregler 32 ist mit jedem Pufferbehälter 14 des Systems 10 gekoppelt. Zwischen dem Flüssigkeitsdurchflussregler 32 und dem Pufferbehälter 14 einer Speichereinheit 18, 20 sind jeweilige Absperrventile 34a, 34b angeordnet.

Mindestens eines der Absperrventile 34a, 34b weist eine Konfiguration auf, die "normal offen" ist. Das bedeutet, dass das jeweilige Absperrventil 34a, 34b im Normalfall, ohne die Beaufschlagung mit einem entsprechenden Stellsignal, offen ist. Das Stellsignal ist notwendig, um das Absperrventil 34a, 34b zu schließen. Generell können auch alle Absperrventile 34a, 34b als "normal offen" ausgebildet sein.

Der Flüssigkeitsdurchflussregler 32 ist eingerichtet, um einen vorbestimmten Volumenstrom des Sterilisationsmediums 12 stromabwärts bereitzustellen.

Vorliegend umfasst das System 10 zusätzlich eine generell optionale Zweistoffdüse 36, die stromabwärts von dem Flüssigkeitsdurchflussregler 32 angeordnet ist. Der von dem Flüssigkeitsdurchflussregler 32 bereitgestellte Volumenstrom des Sterilisationsmediums 12 wird mittels der Zweistoffdüse 36 anhand eines Trägermediums zerstäubt. Das zerstäubte Gemisch aus Trägermedium und Sterilisationsmedium 12 wird anschließend am Aerosolausgang 38 für eine gewünschte Anwendung bereitgestellt.

Das System 10 umfasst ferner eine Druckmedienquelle 40, hier einen Druckmedieneingang, anhand der das Druckmedium 41 (hier Druckluft) für das System 10 bereitgestellt wird.

Gemäß dieser Ausführungsform ist ferner eine Wartungseinheit 42 vorgesehen, die stromabwärts bezüglich der Druckmedienquelle 40 angeordnet ist. Die Wartungseinheit 42 umfasst vorliegend einen Kugelhahn, eine Filtervorrichtung und eine Druckregelvorrichtung, um die Bereitstellung des Druckmediums 41 innerhalb des Systems 10 bedarfsgerecht steuern zu können.

Zudem wird das Druckmedium 41 vorliegend zusätzlich auch zum Betrieb zumindest der Absperrventile 26 genutzt. Das bedeutet, dass die Absperrventile 26 pneumatisch betrieben sind.

Zur entsprechenden Steuerung der Absperrventile 26 sind daher Vorsteuerventile 44 vorgesehen, die vorliegend als 3/2-Wegeventile ausgebildet sind und mit der Druckmedienquelle 40 gekoppelt sind. Anhand der Vorsteuerventile 44 kann ein entsprechender Steuerdruck für die Absperrventile 26 bereitgestellt werden, sodass die Schaltstellung des jeweiligen Absperrventils 26 beeinflusst werden kann.

Zusätzlich weist das System 10 gemäß dieser Ausführungsform einen mechanischen Druckregler 46 (Druckregelvorrichtung) und ein Manometer 48 auf, die mit der Druckmedienquelle 40 gekoppelt sind. Der Druckregler 46 kann auch elektronisch sein. Stromabwärts vom mechanischen Druckregler 46 sind 3/2-Wege Bedruckungsventile 50 für jede Speichereinheit 18, 20 angeordnet, die vorliegend ein integrales Proportionalventil umfassen. Ausgehend von den 3/2-Wege Bedruckungsventilen 50 ist die Druckmedienquelle 40 mit den Pufferbehälter 14 gekoppelt, wobei zwischen dem jeweiligen 3/2-Wege Bedruckungsventil 50 und dem jeweiligen Pufferbehälter 14 zusätzlich jeweils ein Rückschlagventil 58 angeordnet ist. Das Rückschlagventil 58 verhindert einen Rückfluss eines Gases oder einer Flüssigkeit aus dem Pufferbehälter 14 in Richtung der Druckmedienquelle 40.

Gemäß dieser Ausführungsform wird das Druckmedium 41 nicht nur genutzt, um eine Druckpolster in den Pufferbehältern 14 zu erzeugen, sondern auch als Fördermedium bei der Zerstäubung anhand der Zweistoffdüse 36. Daher ist mit der Druckmedienquelle 40 auch ein Massendurchflussregler 52 gekoppelt. Stromabwärts vom Massendurchflussregler 52 ist ein Drucktransmitter 54 angeordnet, bevor das Druckmedium 41 vorliegend als Fördermedium für die Zweistoffdüse 36 bereitgestellt wird.

Die Wartungseinheit 42, die Vorsteuerventile 44, der mechanische Druckregler 46, das Manometer 48, die 3/2-Wege Bedruckungsventile 50, der Massendurchflussregler 52 und der Drucktransmitter 54 können generell als Teil der Druckmedienversorgungsvorrichtung 56 angesehen werden.

Jeder Pufferbehälter 14 weist einen Füllstandsmesser 59 auf, der eingerichtet ist, um einen Füllstand des Sterilisationsmediums 12 innerhalb des Pufferbehälters 14 zu erfassen.

Jeder Pufferbehälter 14 ist zudem mit einem Manometer 60 gekoppelt, das optional einen Drucktransmitter aufweist. Ferner ist mit jedem Pufferbehälter 14 ein mechanisches Druckentlastungsventil 62 gekoppelt, das eine Sicherheitsfunktionalität gewährleistet. Zusätzlich ist auch jeweils ein elektrisches Druckentlastungsventil 64 vorgesehen, wobei das mechanische Druckentlastungsventil 62 dazu parallel angeordnet ist. Stromabwärts vom mechanischen Druckentlastungsventil 62 und dem elektrischen Druckentlastungsventil 64 ist der Pufferbehälter 14 wiederum mit der Drainage 28 gekoppelt.

Außer den Absperrventilen 26 können generell auch die Absperrventile 30 pneumatisch betrieben sein.

Zusätzlich weist das System eine hier nicht dargestellte Steuervorrichtung auf, die mit den Ventilen gekoppelt ist, um für einen jeweiligen Pufferbehälter 14 den Füllmodus oder den Sprühmodus einzustellen. Das bedeutet, dass die Steuervorrichtung entsprechende Stellsignale bereitstellen kann, um damit Ventile zu öffnen oder zu schließen. Zusätzlich ist die Steuervorrichtung im Allgemeinen auch mit weiteren Komponenten des Systems 10 gekoppelt, beispielsweise den Füllstandmessern 59, den Manometern 48, 60, dem Flüssigkeitsdurchflussregler 32, der Zweistoffdüse 36, dem Massendurchflussregler 52 und dem Drucktransmitter 54.

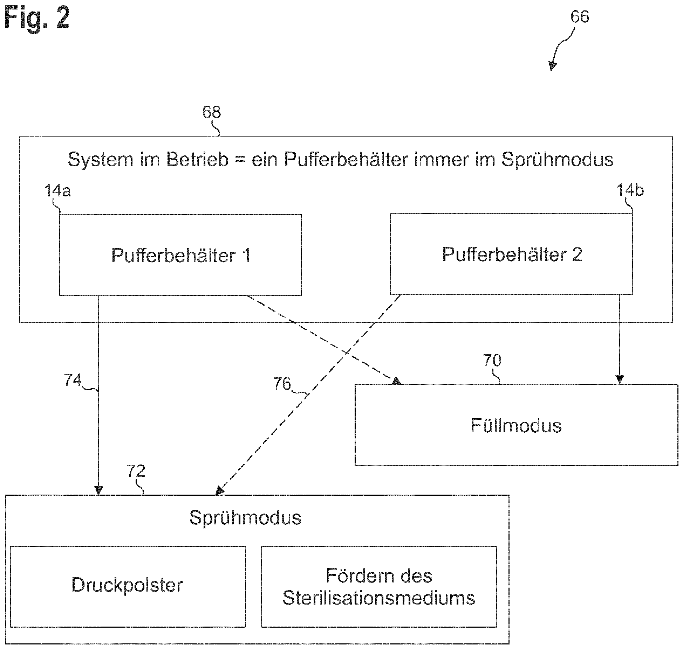

Fig. 2 zeigt eine schematische Darstellung eines erfindungsgemäßen Verfahrens 66 zur Bereitstellung eines Sterilisationsmediums 12. Das Verfahren 66 wird dabei mit dem zuvor beschriebenen System 10 ausgeführt.

Das System 10 umfasst wie bereits beschrieben einen ersten Pufferbehälter 14a, und einen zweiten Pufferbehälter 14b. Während des Betriebs 68 des Systems 10, ist für zumindest einen Pufferbehälter 14 immer der Sprühmodus 72 eingestellt. Dazu wird die Steuervorrichtung genutzt.

So kann im Sprühmodus 72 insbesondere anhand der Druckmedienquelle 40 und der 3/2-Wege Bedruckungsventile 50 das Druckmedium 41 in einen Pufferbehälter 14 eingeleitet werden, um oberhalb einer Menge des Sterilisationsmediums 12 ein Druckpolster zu gewährleisten. Der Druck des Druckpolsters wird dabei durch die Steuervorrichtung geregelt, die anhand des Druckreglers 46, des 3/2-Wege Bedruckungsventils 50 und eines optionalen Proportionalventils einen entsprechenden Regelkreis gewährleisten kann.

Da der jeweilige Pufferbehälter 14, sofern für ihn der Sprühmodus eingestellt ist, fluidisch bezüglich des Sterilisationsmediums 12 mit dem Flüssigkeitsdurchflussregler 32 gekoppelt ist (das jeweilige Absperrventil 34 ist dann offen), wird durch das bereitgestellte Druckpolster eine Förderung des Sterilisationsmediums 12 ermöglicht.

Für den anderen Pufferbehälter 14 ist dann der Füllmodus 70 eingestellt. Im Füllmodus 70 ist für den jeweiligen Pufferbehälter 14 das Absperrventil 34 geschlossen. Ferner ist auch das jeweils zugeordnete 3/2-Wege Bedruckungsventil 50 geschlossen. Hingegen ist das Absperrventil 26 geöffnet. Der Pufferbehälter 14 ist dann fluidisch mit der Sterilisationsmedienquelle 22 gekoppelt. In diesem Fall ist zusätzlich für den jeweiligen Pufferbehälter 14 zumindest das elektrische Druckentlastungsventil 64 geöffnet. Folglich herrscht in dem Pufferbehälter 14 Atmosphärendruck. Der Füllvorgang kann dann vorteilhafter Weise gegen Atmosphärendruck erfolgen.

In einer ersten Konfiguration (durchgezogene Pfeile 74) ist für den ersten Pufferbehälter 14a der Sprühmodus 72 eingestellt und für den zweiten Pufferbehälter 14b der Füllmodus 70. Fällt der Pegel des Sterilisationsmediums im ersten Pufferbehälter 14a unter einen vordefinierten Schwellwert, so wird die Einstellung geändert.

Gemäß der zweiten Konfiguration (gestrichelte Pfeile 76) wird dann für den zweiten Pufferbehälter 14b der Sprühmodus 72 eingestellt und für den ersten Pufferbehälter 14a der Füllmodus.

So kann gewährleistet werden, dass im Betrieb 68 des Systems 10 immer für zumindest einen Pufferbehälter 14 der Sprühmodus 72 eingestellt ist. Dazu wird die bereits erwähnte Steuervorrichtung genutzt, anhand der die Ventile bedarfsgerecht gesteuert und geschaltet werden können.

In der vorliegenden Anmeldung kann auf Mengen und Zahlen Bezug genommen werden. Sofern nicht ausdrücklich angegeben, sind solche Mengen und Zahlen nicht als einschränkend zu betrachten, sondern als Beispiele für die möglichen Mengen oder Zahlen im Zusammenhang mit der vorliegenden Anmeldung. In diesem Zusammenhang kann in der vorliegenden Anmeldung auch der Begriff "Mehrzahl" verwendet werden, um auf eine Menge oder Zahl zu verweisen. In diesem Zusammenhang ist mit dem Begriff "Mehrzahl" jede Zahl gemeint, die größer als eins ist, z. B. zwei, drei, vier, fünf, usw. Die Begriffe "etwa", "ungefähr", "nahe" usw. bedeuten plus oder minus 5 % des angegebenen Wertes.

## Patentansprüche

1. System (10) zur Förderung eines Sterilisationsmediums (12), umfassend zumindest zwei schaltungstechnisch parallel angeordnete Pufferbehälter (14) zur Speicherung des Sterilisationsmediums (12), zumindest einen Flüssigkeitsdurchflussregler (32), mit dem die Pufferbehälter (14) jeweils fluidisch gekoppelt sind, und einer Druckmedienversorgungsvorrichtung (56), die eine Druckmediumquelle (40) mit den Pufferbehältern (14) koppelt und eingerichtet ist, die Pufferbehälter (14) mit einem Druckmedium (41) zu beaufschlagen, wobei das System (10) derart ausgebildet ist, dass für jeden Pufferbehälter (14) ein Füllmodus und ein Sprühmodus einstellbar sind, wobei im Füllmodus der entsprechend geschaltete Pufferbehälter (14) mit dem Sterilisationsmedium (12) aus einer Versorgungsvorrichtung (22) befüllbar ist und im Sprühmodus fluidisch mit dem Flüssigkeitsdurchflussregler (32) gekoppelt ist und das Sterilisationsmedium (12) aus diesem Pufferbehälter (14) in Richtung des Flüssigkeitsdurchflussreglers (32) basierend auf einem von dem Druckmedium (41) ausgeübten Druck förderbar ist, und wobei die Pufferbehälter (14) wechselweise schaltbar sind, so dass während des Sprühmodus des einen Behälters der andere oder ein anderer Pufferbehälter (14) im Füllmodus ist.

2. System (10) nach Anspruch 1, **dadurch gekennzeichnet, dass** im Pufferbehälter (14), für den der Füllmodus eingestellt ist, Atmosphärendruck herrscht.

3. System (10) nach Anspruch 1 oder 2, **dadurch gekennzeichnet, dass** jeder Pufferbehälter (14) jeweils mit einer mechanischen oder elektronischen Druckmessvorrichtung (60) gekoppelt ist, um den Fluiddruck im Pufferbehälter (14) zu bestimmen.

4. System (10) nach einem der vorherigen Ansprüche, **dadurch gekennzeichnet, dass** jeder Pufferbehälter (14) zumindest einen Füllstandsmesser (59) aufweist.

5. System (10) nach einem der vorherigen Ansprüche, **dadurch gekennzeichnet, dass** das System (10) ferner eine mechanische oder elektronische Druckregelungsvorrichtung (46) aufweist, die zumindest mit den Pufferbehältern (14) und der Druckmedienversorgungsvorrichtung (56) gekoppelt und eingerichtet ist, jeden Pufferbehälter (14) mit einem vordefinierten Druck basierend auf dem Druckmedium (41) zu beaufschlagen.

6. System (10) nach Anspruch 5, **dadurch gekennzeichnet, dass** das System (10) zwischen der Druckregelungsvorrichtung (46) und den Pufferbehältern (14) für jeden Pufferbehälter (14) ein Proportionalventil aufweist.

7. System (10) nach einem der vorherigen Ansprüche, **dadurch gekennzeichnet, dass** jeder Pufferbehälter (14) jeweils mit zumindest einem Druckentlastungsventil (64) gekoppelt ist.

8. System (10) nach einem der vorherigen Ansprüche, **dadurch gekennzeichnet, dass** zwischen jedem Pufferbehälter (14) und der Druckmedienversorgungsvorrichtung (56) ein Rückschlagventil (58) derart angeordnet ist, dass ein Rückfluss vom Pufferbehälter (14) in die Druckmedienversorgungsvorrichtung (56) unterbunden wird.

9. System (10) nach einem der vorherigen Ansprüche, **dadurch gekennzeichnet, dass** das System (10) ferner zumindest eine Zweistoffdüse (36) aufweist, die ausgehend von dem Flüssigkeitsdurchflussregler (32) mit dem Sterilisationsmedium (12) und zusätzlich mit einem Fördermedium beaufschlagbar ist, und eingerichtet ist, das Sterilisationsmedium (12) durch das Fördermedium zu zerstäuben.

10. System (10) nach einem der vorherigen Ansprüche, **dadurch gekennzeichnet, dass** das System (10) für jeden Pufferbehälter (14) ferner eine Spülvorrichtung aufweist, die eingerichtet ist, um den jeweiligen Pufferbehälter (14) mit einem Reinigungsmittel zu spülen.

11. System (10) nach einem der vorherigen Ansprüche, **dadurch gekennzeichnet, dass** jeder Pufferbehälter (14) eine Auslassleitung hat, die zu einer gemeinsamen Leitung zum Flüssigkeitsdurchflussregler (32) führt, wobei in den Auslassleitungen Absperrventile (34) vorgesehen sind, die im Füllmodus geschlossen sind.

12. Verfahren zur Bereitstellung eines Sterilisationsmediums (12) anhand eines Systems (10) nach einem der vorherigen Ansprüche,
wobei, sofern für einen Pufferbehälter (14) der Füllmodus eingestellt ist, dieser Pufferbehälter (14) mit dem Sterilisationsmedium (12) aus einer Versorgungsvorrichtung (22) befüllt wird,
wobei, sofern für einen Pufferbehälter (14) der Sprühmodus eingestellt ist, dieser Pufferbehälter (14) mit dem Druckmedium (41) ausgehend von der Druckmedienversorgungsvorrichtung (56) derart beaufschlagt wird, dass ein Druck im Pufferbehälter (14) erzeugt wird, dieser Pufferbehälter (14) mit dem Flüssigkeitsdurchflussregler (32) fluidisch gekoppelt ist und basierend auf dem durch das Druckmedium (41) im Pufferbehälter (14) erzeugten Druck Sterilisationsmedium (12) aus diesem Pufferbehälter (14) in Richtung des Flüssigkeitsdurchflussreglers (32) gefördert wird, und
wobei das System (10) während des Betriebs des Systems (10) derart schaltbar ist, dass immer für zumindest einen Pufferbehälter (14) der Sprühmodus einstellbar ist.
